(19) Europäisches Patentamt / European Patent Office / Office européen des brevets

(11) **EP 4 137 587 A1**

(12) **EUROPEAN PATENT APPLICATION**

(43) Date of publication:
**22.02.2023 Bulletin 2023/08**

(21) Application number: **22183165.4**

(22) Date of filing: **05.07.2022**

(51) International Patent Classification (IPC):
***C12Q 1/6888*** (2018.01)

(52) Cooperative Patent Classification (CPC):
**C12Q 1/6888**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.07.2021 IT 202100017717**

(71) Applicant: **Ingeno S.r.l.**
**20129 Milano (MI) (IT)**

(72) Inventor: **Garoia, Flavio**
**20129 Milano MI (IT)**

(74) Representative: **Perani & Partners S.p.A.**
**Piazza Armando Diaz, 7**
**20123 Milano (IT)**

Remarks:
•The complete document including Reference Table(s) and the Sequence Listing(s) can be downloaded from the EPO website
•A request for correction of the sequence listing has been filed pursuant to Rule 139 EPC. A decision on the request will be taken during the proceedings before the Examining Division (Guidelines for Examination in the EPO, A-V, 3.).

(54) **SCREENING METHOD OF A MITE OF DEMODEX SPP**

(57) The object of the invention relates to a screening method of mite species of *Demodex spp* comprising the steps of: a) preparing a sample of biological material for which it is intended to verify the presence of *Demodex spp* genetic material; b) extracting the genetic material from the prepared sample of biological material; c) conducting a quantitative PCR analysis of the extracted genetic material, using a pair of primers synthesised for the amplification of a pre-determined sequence of *Demodex spp* genetic material, wherein the pre-determined sequence of *Demodex spp* genetic material is a nuclear DNA or mitochondrial DNA sequence of *Demodex spp.*

| Variable | Obs. | Min. | Max. | Average | Std Deviat. |
|---|---|---|---|---|---|
| Demodex + | 6 | 19,370 | 31,110 | 26,147 | 4,687 |
| Demodex - | 12 | 30,605 | 42,585 | 39,853 | 3,366 |

Figure 1

EP 4 137 587 A1

Description

FIELD OF THE INVENTION

The field of the present invention concerns a method for screening an obligate human ecto-parasite, primers suitable for the identification thereof and a diagnostic kit useful for these purposes.

BACKGROUND

The mite of the *Demodex* species is an obligate human ecto-parasite. The elective site of this type of mite is generally represented by hair follicles and sebaceous glands. Two species have been described in humans: *Demodex folliculorum,* predominantly localised in the hair follicle, and *Demodex brevis,* with a more internal localisation, in the lumen of the sebaceous glands [1].

These parasites, through their limbs and palpi, scratch and flake the inner wall of the hair follicle and, through their chelicerae, puncture and empty the cytoplasm of the cells of the inner wall of the sebaceous glands, causing intense sebaceous secretion.

The buccal apparatus of the *Demodex* possesses two perforating stylets, through which the mite punctures the cytoplasm of the cells, from inside the sebaceous glands, causing hypersecretion thereof [2]. The salivary glands allow a pre-oral digestion that fluidizes fats, through the release of a lipase, allowing them to enter the alimentary canal, which ends at the caecum. The mites of the species *Demodex* do not have an anal opening and therefore do not emit their own faeces through the terminal part of the intestine. It appears that these mites can accumulate, in the form of cytoplasmic crystals, in the cells of the anterior tract of the intestine, those excretion products that cannot be eliminated through the cuticle or gnathostome [3].

The reproduction originates in the follicular ostium where copulation takes place, after which the fecundated females re-enter the sebaceous glands and within twelve hours begin oviposition. The larva hatches 60 hours later and feeds immediately and continuously, pricking the cells of the pilosebaceous complex, and after a successive series of stages it moults into an adult after about 14.5 days [4].

The female remains close to the follicular ostium until copulation, after which she reenters the sebaceous glands to lay her eggs and, after about 120 hours, returns to the surface where she dies, mixing with the sebaceous secretions and occluding, together with them, the follicle itself. Principato [5] showed that, during the summer period, the sebum of people suffering from demodicosis is richer in eggs than at other times of the year and that this material very easily contaminates objects of use such as glasses or towels and transmits the parasite's eggs "by imprint" to children, who may already be positive for the presence of *Demodex spp* at six months.

Infestation by *Demodex spp* is common; the prevalence in healthy adults varies between 23% and 100%. The number of mites *Demodex spp* increases with age. The prevalence of the infestation is higher in the 20-30 age group, when the sebum secretion rate is at its maximum, and is also found in high density in the adult and elderly population [6, 7].

Infestation by *Demodex spp* usually remains asymptomatic and may only play a pathogenic role if present in high densities or in subjective situations of increased sensitivity such as in the presence of disorders of the immune system. The presence of a high density of this mite can therefore be an aetiological factor involved in a wide variety of dermatoses.

Numerous studies have reported a high density of *Demodex spp* in patients with rosacea [8, 9] or presenting non-specific facial symptoms such as facial pruritus with or without erythema, seborrhoeic dermatitis, lesions similar to perioral and papulo-pustular and/or acneiform dermatitis, facial erythema, dryness, scaling and roughness [10, 11, 12, 13].

The density of *Demodex spp* in the scalp has been related to the risk of baldness in numerous scientific studies [14]. Although baldness (androgenetic alopecia) is a problem primarily due to hormonal factors that act concurrently with a genetic predisposition, it is likely that inflammatory processes deriving from the presence of *Demodex spp* in high density at the scalp level may aggravate the clinical picture by accelerating the progression of baldness [15, 16].

Therefore, assessing the density of *Demodex spp* in the skin and scalp in the presence of dermatosis or excessive hair loss can help the physician in early diagnosis and appropriate and timely management of the disorder.

*Problem of known art*

The presence of *Demodex spp* infesting humans is not easily detectable in histological preparations; therefore, the skin surface biopsy (SSB) technique with cyanoacrylic adhesion is a commonly used method to measure the density of *Demodex spp* [17], and involves collecting the superficial part of the stratum corneum and the contents of the pilosebaceous follicle [18], although it has been shown not to be fully efficient [19]. Other sampling methods used in assessing the presence of *Demodex spp* by microscopy comprise the use of invasive sampling methods, such as adhesive strips, skin scrapings, hair removal and punch biopsies [20].

A traditional technique is based on the direct microscopic examination (*DME*)*,* and involves the use of a comedo extractor. This technique requires samples obtained from papules or follicular pustules by squeezing; moreover, it is notoriously invasive and painful [21].

Therefore, all these methods known in the state of the art have limitations related to the mode of execution (see microscopy) and the invasiveness of the collection method.

## SUMMARY OF THE INVENTION

A first object of the present invention is a screening method of mite species of *Demodex spp* comprising the steps of:

> a) preparing a sample of biological material for which it is intended to verify the presence of *Demodex spp* genetic material;
>
> b) extracting the genetic material from the prepared sample of biological material;
>
> c) conducting a quantitative PCR analysis of the extracted genetic material, using a pair of primers synthesised for the amplification of a pre-determined sequence of *Demodex spp* genetic material, wherein the pre-determined sequence of *Demodex spp* genetic material is a nuclear DNA or mitochondrial DNA sequence of *Demodex spp.*

A further object of the invention is a pair of primers suitable for use in quantitative PCR analysis, preferably RT-PCR, according to the method of the invention.

Finally, another object of the invention is a diagnostic kit, suitable for carrying out the screening method according to the invention, comprising: a sterile medical swab; a sterile test tube and possibly a buffer for immersion of the swab after sampling; a pair of primers for amplification of a sequence of *Demodex spp* genetic material, said pair of primers being the object of the invention.

### *Advantages of the invention*

The screening method developed by the Applicant provides a means for a reliable detection of the density of the mite in the scalp and skin.

The method is characterised by a higher sensitivity than traditional methods: although material from the surface of the skin and scalp is collected, the quantitative PCR has a very high sensitivity in detecting DNA that could originate both from any mites collected, and from the residue of tissues deriving the life cycle of *Demodex* (tissue fragments deriving from dead individuals, eggs, etc.) that are brought to the surface with sebaceous secretions. Advantageously, the Applicant was able to design selective sequences of primers for both species of *Demodex* infesting humans, namely *D. folliculorum* and *D. brevis.*

The method is rapid, painless and versatile for the detection of *Demodex spp,* preferably at the skin and scalp level.

The screening method developed represents a sensitive, non-invasive, easy-to-perform and cost-effective test in terms of materials, sampling and evaluation of the result, and allows its use both directly by the patient and by healthcare professionals.

To this end, the Applicant has also developed a diagnostic kit that can be used by both patients and by more experienced users.

As shown in the experimental section, the method developed by the Applicant can be correlated with traditional counting methods. In particular, it was demonstrated that the threshold cycle (Ct) value of the PCR analysis detected in the samples that tested positive in the microscopic analysis (standard counting method) was significantly higher than the samples that tested negative (and vice versa), thus signalling a good specificity of the screening method.

## DESCRIPTION OF THE DRAWINGS

> Figure 1 - Comparison graph of the Ct values for the group of samples that tested positive in the presence of *Demodex spp* (indicated by "Demodex spp +") and the group of samples tested negative in the presence of *Demodex spp* (indicated by "Demodex spp -") (see Example 1);
>
> Figure 2 - Correlation graph between Ct measurement by the molecular screening method according to the invention (indicated with "Demodex Ct"), and the measurement by microscopic counting according to the traditional method using the comedo extractor (indicated with "Demodex N.") (see Example 1).

## DETAILED DESCRIPTION OF THE INVENTION

The Applicant describes the objects of the invention below.

### *Screening method of a mite Demodex spp*

Screening method of a mite *Demodex spp* means a qualitative-quantitative analysis of *Demodex spp* in a biological material.

Biological material means one or more tissues and/or tissue derivatives of animals or humans, such as, for example, skin tissue (skin) or scalp, body hair or hair.

Preferably, the species of *Demodex spp* identifiable by the method of the invention comprise or consist of: *Demodex folliculorum* and/or *Demodex brevis.*

### *Step of preparation of biological material (a)*

Preferably, the step of preparation of biological material (a) comprises the sub-steps of:

a1) identifying a collection site, said collection site preferably having an area comprised between 0.5 and 2.5 cm$^2$, preferably equal to 1 cm$^2$;

a2) collecting a sample of the biological material contained in the area of the identified collection site using a sampling medium.

Preferably, the collection medium is a sterile medical (or laboratory) swab preferably comprising a wooden or plastic stem and a cotton tip.

The collection site is preferably a site located at the level of the skin tissue (skin) and/or scalp.

The biological sample can therefore be a skin and/or scalp sample. Preferably, the biological sample contains portions of skin and/or scalp deriving from the flaking of the stratum corneum. Still preferably, when the biological sample consists of skin (not of scalp), the skin tissue is that of the face, preferably of the sebaceous areas of the face such as forehead, nose, chin.

Preferably, the biological material collection step a2) comprises the following sub-steps of:

a2.1) preparing a sterile medical swab,
a2.2) rubbing the sterile medical swab over the sampling site to obtain a sample of biological material,
a2.3) depositing the medical swab with the sample of biological material in a sterile test tube, said test tube optionally containing a buffer.

The step of rubbing the medical swab (a2.2) preferably involves rubbing the swab vigorously for at least 40 seconds, preferably for at least 20 seconds, at the collection point.

Preferably, when the collection site is the skin, the swab is rubbed onto the skin, rotating it, for at least 40 seconds, preferably for at least 20 seconds.

Preferably, when the collection site is the scalp, the swab is rubbed, rotated, for at least 40 seconds, preferably for at least 20 seconds.

The step of depositing the swab in a sterile test tube (a.2.3) optionally containing buffer advantageously allows to preserve the biological sample collected for subsequent analysis, such as, for example, the step of extracting the genetic material b).

Examples of buffers useful for this purpose are for example: TE buffer (10 mM Tris·HCl and 1 mM EDTA), Tris-acetate-EDTA, Tris-glycine and Tris-borate-EDTA. The buffers may also contain additives such as urea and SDS.

It should be noted that the step of preparing a biological sample (a) conducted in accordance with the invention, is advantageously non-invasive and painless; moreover, the sample thus collected can be stored at room temperature and does not require any special precautions for shipment (e.g. refrigerated transport).

### *Step of extraction of the genetic material from the collection of prepared biological material (b)*

Preferably, the extraction of genetic material from the sample of biological material can take place using any method known to the state of the art.

Illustrative and non-limiting examples of DNA extraction techniques suitable for the purposes of the invention are: organic extraction, extraction with silica membranes or substrates, magnetic separation, extraction with anion exchange membranes.

For example, for the purposes of the invention, the genetic material was extracted using a silica membrane extraction technique (GeneJET Genomic DNA Purification Kit, Thermofisher Scientific, Maryland USA).

*Step of conducting a PCR of the extracted genetic material (c)*

The analysis of the genetic material extracted from the sample of biological material is performed by quantitative PCR (or q-PCR) analysis, preferably Real-Time PCR (or RT-PCR), preferably Real-Time Fluorescence PCR.

As is well known, the PCR analysis involves a succession of amplification cycles; each amplification cycle comprises the following steps: denaturation (opening) of the DNA; pairing of the primers (annealing); extension of the primers (elongation).

According to a preferred embodiment, the PCR analysis according to the method of the invention has a threshold cycle (Ct) comprised between 18 and 40, preferably comprised between 19 and 38.

For the purposes of the present invention, a "threshold cycle" ("Ct") is the point at which the amplification of the target of a sequence of genetic material, preferably DNA, rises above the background, as indicated by a fluorescence signal. In other words, the Ct value indicates how many PCR multiplication cycles must be performed to detect the presence of *Demodex spp* genetic material. In particular, the Ct value is inversely proportional to the amount of the investigated sequence; therefore, the higher the Ct value, the lower the presence of *Demodex spp* in the sample analysed.

For the purposes of this invention, a Ct value < about 37.65 indicates positivity to *Demodex spp.* Conversely, a Ct value > about 37.65 indicates negativity to *Demodex spp.*

Performing the amplification cycle requires a pair of primers complementary to the pre-determined sequence of *Demodex spp* genetic material, said pre-determined sequence being a nuclear or mitochondrial DNA sequence of *Demodex spp.*

Preferably, the method object of the invention comprises, prior to the step of conducting the PCR, a step of synthesising a pair of primers. For a description of the synthesis process, see below in the document.

Preferably, the step of synthesising the primers comprises the following sub-steps:

- identifying one or more nuclear or mitochondrial DNA sequences of *Demodex spp* on the basis of which to synthesise complementary primers;

- identifying the length of the primers to be synthesised (and thus of the amplicon to be obtained);

- identifying the Melting temperature (Tm) of the primers to be designed, where "melting temperature" (Tm) refers to the temperature at which 50% of the DNA is unwound in the form of a single strand.

Preferably, the nuclear or mitochondrial DNA sequence *of Demodex spp* is a homologous sequence, i.e. a sequence common to two or more species of the *Demodex spp* family. Still preferably, the homologous sequence is of the 16S mitochondrial gene of *Demodex spp.*

The pairs of primers can be designed by hand or using special programs (software) that can be installed on the computer or are accessible via the web. An exemplary and non-exhaustive list of programs that can be used as a web service is as follows:

- Primer-Blast: https://www.ncbi.nlm.nih.gov/tools/primer-blast/;

- Primer3: http://biotools.umassmed.edu/bioapps/primer3; http://www.cgi; or http://frodo.wi.mit.edu/;

- GeneFisher: http://bibiserv.techfak.uni-bielefeld.de/genefisher2;

- Oligonucleotide calculator: http://www.basic.northwestern.edu/biotools/oligocalc.html.

By way of example, for the purposes of the invention, Primer-Blast was used.

In general, these softwares are based on algorithms that are able to identify, within the target sequences (e.g. a plurality of gene sequences of *Demodex spp* DNA), the most stable regions for the annealing (alignment) of the primers. In fact, on the basis of the nucleotide sequence, the regions of the DNA can ensure a more or less stable binding of the primer (the more stable the binding, the more efficient the PCR). On the basis of the data provided during the primers synthesis step (target gene sequences, efficiency parameters and amplified length, in terms of number of bases), the program automatically processes a series of possible primers (see Example 2). Subsequently, it is necessary that the primers have to be tested in the laboratory to verify that the theoretical parameters are actually respected even in real amplification.

For the purposes of the invention, an efficiency parameter means one (or more) parameters defined by algorithms, which take into account, in particular, the stability of the binding of the primer to the target DNA. For example, the greater the stability, preferably at the level of the region 3', the greater the efficiency and the specificity of the amplification.

Preferably, the pre-determined sequence of *Demodex spp* genetic material (i.e. the sequence of genetic material on the basis of which the primers are to be designed) is a sequence of a *Demodex spp* gene preferably chosen from the group consisting of: 16S mitochondrial gene, CYTB (mitochondrial), 18S (mitochondrial), 28S (mitochondrial), COI (mitochondrial), actin (nuclear), troponin (nuclear), alpha-tubulin (nuclear), myosin (nuclear).

Still preferably, the pre-determined sequence of genetic material of *Demodex spp* is a homologous sequence of the 16S mitochondrial gene of *Demodex spp.*

According to a preferred embodiment, the length of each of the primers of the pair is characterized by a length comprised between 100 and 220 bp, preferably between 100 and 218 bp, preferably between 120 and 216 bp, preferably between 150 and 216 bp, preferably between 159 and 216 bp, preferably equal to 159 bp, 162 bp, 163 bp, 165 bp, 166 bp, 168 bp or 216 bp. Accordingly, the length of the amplicon to be obtained from the PCR analysis according to the method of the invention is preferably comprised between 100 and 220 bp, preferably between 100 and 218 bp, preferably between 120 and 216 bp, preferably between 150 and 216 bp, preferably between 159 and 216 bp, preferably equal to 159 bp, 162 bp, 163 bp, 165 bp, 166 bp, 168 bp or 216 bp.

The Applicant considers that, in the above-mentioned ranges, the amplified ones are more stable and less likely to form secondary structures that can then give rise to artifacts that would interfere with the correct quantification.

The melting temperature (Tm) of the primers suitable for the purposes of the invention is preferably > 55°C, preferably comprised between about 55.00°C and 60.00°C, preferably between about 58.50°C and 59.90°C, preferably between about 58.65°C and 59.87°C.

The heating of the biological sample comprising genetic material is functional to the breaking of the hydrogen bonds that hold the two strands of DNA paired. The separation caused by the instability of the bonds between the complementary bases is, in fact, indicated by the term "fusion".

The denaturation step of the PCR is then conducted by raising the sample temperature to the melting temperature (Tm). The heating of the biological sample comprising genetic material is functional to the breaking of the hydrogen bonds that hold the two strands of DNA paired. The separation caused by the instability of the bonds between the complementary bases is, in fact, indicated by the term "fusion".

For the purposes of the present invention, heating for denaturation is preferably conducted for a period of time of about 10 seconds.

Still preferably, the pair of primers is characterized by a percentage content in Guanine (G) + Cytosine (C) (GC%) preferably comprised between 45% and 60%, preferably between 55% and 59%, preferably comprised between 57% and 58%.

It should be noted that the G+C content, as well as the length of the primers, influence the determination of the melting temperature (Tm), as well as the annealing temperature (Ta), i.e. the pairing temperature of the primers to the sequence of reference genetic material.

An empirical formula that can be used to calculate Tm, when the primers have an average length of 20 bases (as in the case of the invention), is as follows:

$$Tm = [4(G + C) + 2(A + T)]\,°C$$

wherein G, C, A and T indicate the number of nucleotides containing the nitrogenous bases guanine, cytosine, adenine or thymine. If the two primers have different Tm, preferably the lower one of the two is taken as the Tm.

Preferably, the primers pairing step is conducted at the annealing temperature (Ta) which, for the purposes of the invention, is preferably comprised between 58°C and 85°C, preferably between 60°C and 80°C, preferably between 60°C and 70°C, and preferably between 60°C and 63°C.

For the purposes of the present invention, the heating for pairing is preferably conducted for a period of time comprised between about 10 and 40 seconds, preferably of about 30 seconds.

According to a preferred embodiment, the step of extension (elongation) of the primers is carried out by the enzyme DNA Polymerase, which adds nucleotides in the 5'-3' direction.

The extension step is preferably conducted at a temperature preferably comprised between 58°C and 85°C, preferably between 60°C and 80°C, preferably between 60°C and 70°C, preferably between 60°C and 63°C, preferably 60°C. Furthermore, the duration of the extension step is comprised between about 10 and 40 seconds, preferably equal to 30 seconds.

For the Real Time-PCR analysis, normally the annealing (pairing) and extension steps are carried out simultaneously; therefore, the operating conditions of temperature and time duration are such that both the annealing and extension steps take place.

Preferably, the extension step is conducted in the presence of a reaction buffer containing the oligonucleotides that the DNA polymerase will add for amplification of the target sequence.

Preferably, a reaction buffer suitable for the purposes of the invention comprises at least 50-200 M of each deoxyribonucleotide (dNTP).

Preferably, the primers extension step is conducted at a temperature comprised between 60°C and 70°C, preferably between 60°C and 65°C.

For the purposes of the present invention, the heating for the extension step of the primers is preferably conducted for a period of time of about 30 seconds.

Using the "traditional" PCR technique or analysis, specific sequences in a DNA or cDNA template can be amplified many thousands to a million times. In traditional PCR (*endpoint*), the detection and the quantification of the amplified sequence is performed *at the end of the* reaction, i.e. after the last PCR cycle, and involves post-PCR analysis such as gel electrophoresis and image analysis. In "real time" (RT-PCR) or quantitative PCR analysis, on the other hand, the PCR product is measured *at each cycle.* This is possible by monitoring the reactions during the exponential amplification step of the reaction, e.g. by using fluorescence methods; in this way, the user can determine the initial amount of the target with great precision. In other words, the quantitative PCR is a technique that makes it possible to amplify a determined genomic region by monitoring, in real time, each individual step of the amplification reaction.

The advantages of real-time RT-PCR include: the possibility to monitor the progress of the PCR reaction while it is taking place in real time; the possibility to precisely measure the amount of amplicon at each cycle, which allows a very accurate quantification of the amount of starting material in the samples; a greater dynamic range of detection; and finally, amplification and detection occur in a single step, eliminating post-PCR manipulations.

Preferably, the Real-Time fluorescence PCR analysis involves the use of SYBR Green, i.e. an aromatic organic compound belonging to the asymmetric cyanines, which has fluorophore activity and acts as a nucleic acid intercalant.

Preferably, the quantitative PCR analysis of the screening method of the invention excludes the use of probes, such as TaqMan-type probes. Preferably, the quantitative PCR analysis of the method of the invention does not follow the TaqMan method (Insect Molecular Genetics (Fourth Edition), An Introduction to Principles and Applications, 2019, Pages 263-314, Chapter 7 - DNA Amplification by the Polymerase Chain Reaction: Providing a Revolutionary Method for All Biologists, Marjorie A.Hoy; doi: 10.1016/B978-0-12-815230-0.00007-8).

*Pair of primers*

The Applicant synthesised a pair of primers, comprising a direct primer (forward or left) and a reverse primer (reverse or right) presented below.

Preferably, the pair of primers is designed for screening the species of *Demodex spp* comprising or consisting of *Demodex folliculorum* and/or *Demodex brevis.*

Preferably, the gene sequence amplified by the pair of primers according to the present invention is a mitochondrial or nuclear DNA sequence of *Demodex spp,* preferably a mitochondrial DNA sequence of *Demodex spp,* preferably a homologous sequence of the mitochondrial DNA of *Demodex spp,* preferably a homologous sequence of the 16S mitochondrial gene of *Demodex spp.*

For the nucleotide sequences SEQ ID. No 12, 13, 14 and 15, the symbol "*N*" indicates a nucleotide base or an abasic site (no nucleotide base). Each *N* is indicated by the position occupied in the nucleotide sequence, counted from left to right in ascending order.

*Direct primer*

Preferably, the direct primer is encoded by the generic sequence NTGCTGCGGTATTTTGACTGNN (SEQ ID No. 12),

wherein *N* in position 1 and *N* in position 21 are independently chosen between: abasic site, or Thymine (T),

wherein *N* in position 22 is an abasic site, or Guanine (G).

Preferably, the direct primer is encoded by a sequence chosen from the group consisting of:

| Sequence (5'-3') | Sequence Number (ID) |
|---|---|
| TGCTGCGGTATTTTGACTGTG | 1 |
| TTGCTGCGGTATTTTGACTG | 2 |

Still preferably, the direct (forward) primer is encoded by the sequence SEQ ID No. 1: 5'-TGCTGCGGTATTTTGACTGTG-3'.

*Reverse primer*

Preferably, the reverse primer is encoded by a sequence chosen from the group consisting of:

- NNTTTGGGGTCTTCTCGTCTTN (SEQ ID No. 13), wherein *N* in position 1, *N* in position 2 and *N* in position 22 are independently chosen from: abasic site, or Thymine (T);

- *NNNGATTTTGGGGTCTTCTCGNNN* (SEQ ID No. 14), wherein *N* in position 1, *N* in position 2, *N* in position 3 are independently chosen from: abasic site, or Adenosine (A); *N* in position 22 and *N* in position 24 are independently chosen from: abasic site, or Thymine (T); *N* in position 23 is an abasic site, or Cytosine (C);

- NGGGGTCTTCTCGTCTTTAGGN (SEQ ID No. 15), wherein *N* in position 1 is an abasic site, or Thymine (T), and *N* in position 22 is an abasic site, or Adenosine (A); and

- AAAATCAATCTTTCCCCCAAA (SEQ ID No. 6).

Preferably, the reverse primer is encoded by a sequence chosen from the group consisting of:

| Sequence (5'-3') | Sequence Number (ID) |
|---|---|
| TTTGGGGTCTTCTCGTCTTT | 3 |
| TTTTGGGGTCTTCTCGTCTT | 4 |
| GGGGTCTTCTCGTCTTTAGGA | 5 |
| AAAATCAATCTTTCCCCCAAA | 6 |
| AAAGATTTTGGGGTCTTCTCG | 7 |
| GATTTTGGGGTCTTCTCGTC | 8 |
| GATTTTGGGGTCTTCTCGTCT | 9 |
| AGATTTTGGGGTCTTCTCGTC | 10 |
| TGGGGTCTTCTCGTCTTTAGG | 11 |

Preferably, the reverse primer is encoded by the sequence SEQ ID No. 11: 5'-TGGGGTCTTCTCGTCTTTAGG-3'.

*Pair of primers*

Preferably, the pair of primers is chosen from the group consisting of:

| No. Pair of primers | Direct primer sequence | Reverse primer sequence |
|---|---|---|
| I | SEQ ID No. 2: TTGCTGCGGTATTTTGACTG | SEQ ID No. 3: TTTGGGGTCTTCTCGTCTTT |
| II | SEQ ID No. 2: TTGCTGCGGTATTTTGACTG | SEQ ID No. 4: TTTTGGGGTCTTCTCGTCTT |
| III | SEQ ID No. 2: | SEQ ID No. 5: |

(continued)

| No. Pair of primers | Direct primer sequence | Reverse primer sequence |
|---|---|---|
| | TTGCTGCGGTATTTTGACTG | GGGGTCTTCTCGTCTTTAGGA |
| IV | SEQ ID No. 2: TTGCTGCGGTATTTTGACTG | SEQ ID No. 6: AAAATCAATCTTTCCCCCAAA |
| V | SEQ ID No. 2: TTGCTGCGGTATTTTGACTG | SEQ ID No. 7: AAAGATTTTGGGGTCTTCTCG |
| VI | SEQ ID No. 2: TTGCTGCGGTATTTTGACTG | SEQ ID No. 8: GATTTTGGGGTCTTCTCGTC |
| VII | SEQ ID No. 2: TTGCTGCGGTATTTTGACTG | SEQ ID No. 9: GATTTTGGGGTCTTCTCGTCT |
| VIII | SEQ ID No. 2: TTGCTGCGGTATTTTGACTG | SEQ ID No. 10: AGATTTTGGGGTCTTCTCGTC |
| IX | SEQ ID No. 1: TGCTGCGGTATTTTGACTGTG | SEQ ID No. 11: TGGGGTCTTCTCGTCTTTAGG |

Preferably, the preferred pair of primers is No. IX.

Such direct and inverse primers are preferably obtained by a process of designing the same which involves the use of two or more gene sequences of mitochondrial or nuclear DNA of *Demodex spp,* and of at least one mitochondrial or nuclear DNA sequence of *Homo Sapiens.*

*Synthesis process of the direct and reverse primers according to the invention*

The process of synthesis (or design) of the primers according to the invention preferably comprises the following steps:

- preparing two or more nuclear or mitochondrial DNA gene sequences of *Demodex spp,* said sequences belonging to the same gene as *Demodex spp*;

- preparing at least one gene sequence of human nuclear or mitochondrial DNA (*Homo Sapiens*)*,* said gene sequence belonging to the human gene corresponding to the chosen *Demodex spp* gene;

- aligning the plurality of nuclear or mitochondrial DNA gene sequences of *Demodex spp,* and identifying sequence homologies between them;

- comparing the nuclear or mitochondrial DNA gene sequences of *Demodex spp* with the at least one gene sequence of *Homo Sapiens*; then discarding the nuclear or mitochondrial DNA gene sequences of *Demodex spp* that have a possible sequence homology with the nuclear or mitochondrial DNA gene sequences of *Homo Sapiens*;

- synthesising a direct primer (forward) and a reverse primer (reverse) on the basis of the sequence homologies identified in the alignment and comparison steps.

As already mentioned, the primers synthesis process can be conducted using the software already mentioned in the chapter on *"Step of conducting a PCR of the extracted genetic material (c)".*

Sequence alignment refers to the process of pairing similar gene regions shared by two or more sequences. The purpose of sequence alignment is to determine whether the compared sequences show sufficient likeness (or similarity) to justify homology inference.

Preferably, for the purposes of the invention, the alignment step can be performed with the aid of software such as Clustal Omega (https://www.ebi.ac.uk/Tools/msa/clustalo/), Unipro UGENE (downloadable from httn://ugene.net/), Clustal X (downloadable from http://www.clustal.org/clustal2/).

In the purposes of the invention, for example, Clustal Omega was used.

Preferably, the two or more DNA sequences of *Demodex spp* prepared in the primers synthesis process are mitochondrial DNA gene sequences, preferably they are sequences of the 16S mitochondrial gene of *Demodex spp.*

The at least one human nuclear or mitochondrial DNA gene sequence (*Homo Sapiens*)*,* is preferably a human mitochondrial DNA gene sequence (*Homo Sapiens*)*,* preferably the human 16S mitochondrial gene sequence (*Homo Sapiens*).

The Applicant considers that the 16S mitochondrial gene has an ideal sequence variability for the purposes of the invention to be able to distinguish different species; at the same time, it is sufficiently conserved within species.

Therefore, all individuals belonging to the same species have a sequence homology greater than 99.9%.

Sequence homology refers to the condition that two sequences are homologous; those sequences that have a common ancestral gene, or share a progenitor, are called homologous. The degree of similarity between two sequences can be measured, whereas homology is a qualitative fact. The similarity of two sequences is a measurable quantity that can be expressed as, for example, percentage identity.

Preferably, the two or more sequences of the 16S mitochondrial gene of *Demodex spp* are chosen from two or more sequences of:

- 16S mitochondrial gene of *Demodex folliculorum,* belonging to the group consisting of

| Definitions | GenBank accession no. | Version |
|---|---|---|
| Df-1 16S ribosomal RNA gene isolated from Demodex folliculorum, partial sequence; mitochondrial. | JF783994 | JF783994.1 |
| Df-S5 16S ribosomal RNA gene isolated from Demodex folliculorum, partial sequence; mitochondrial. | KC917232 | KC917232.1 |
| Df-5 16S ribosomal RNA gene isolated from Demodex folliculorum, partial sequence; mitochondrial. | JF783997 | JF783997.1 |
| Df-S10 16S ribosomal RNA gene isolated from Demodex folliculorum, partial sequence; mitochondrial. | KC917237 | KC917237.1 |
| Df-S6 16S ribosomal RNA gene isolated from Demodex folliculorum, partial sequence; mitochondrial. | KC917233 | KC917233.1 |
| Df-S2 16S ribosomal RNA gene isolated from Demodex folliculorum, partial sequence; mitochondrial. | KC917229 | KC917229.1 |
| Df-4 16S ribosomal RNA gene isolated from Demodex folliculorum, partial sequence; mitochondrial. | JF783996 | JF783996.1 |
| Df-S9 16S ribosomal RNA gene isolated from Demodex folliculorum, partial sequence; mitochondrial. | KC917236 | KC917236.1 |
| Df-S7 16S ribosomal RNA gene isolated from Demodex folliculorum, partial sequence; mitochondrial. | KC917234 | KC917234.1 |
| and mixtures of the above; | | |

and/or

- 16S mitochondrial gene of *Demodex brevis,* belonging to the group consisting of:

| Definitions | GenBank accession no. | Version |
|---|---|---|
| 1 16S ribosomal RNA gene isolated from Demodex folliculorum, partial sequence; mitochondrial. | KX789098 | KX789098.1 |
| 2 16S ribosomal RNA gene isolated from Demodex folliculorum, partial sequence; mitochondrial. | KX789099 | KX789099.1 |
| Db-3 16S ribosomal RNA gene isolated from Demodex folliculorum, partial sequence; mitochondrial. | JF783999 | JF783999.1 |
| Db-S3 16S ribosomal RNA gene isolated from Demodex folliculorum, partial sequence; mitochondrial. | KC917240 | KC917240.1 |
| Db-S1 16S ribosomal RNA gene isolated from Demodex folliculorum, partial sequence; mitochondrial. | KC917238 | KC917238.1 |
| Db-1 16S ribosomal RNA gene isolated from Demodex folliculorum, partial sequence; mitochondrial. | JF783998 | JF783998.1 |
| Db-2 16S ribosomal RNA gene isolated from Demodex folliculorum, partial sequence; mitochondrial. | HQ844220 | HQ844220.1 |

(continued)

| Definitions | GenBank accession no. | Version |
|---|---|---|
| Db-S2 16S ribosomal RNA gene isolated from Demodex folliculorum, partial sequence; mitochondrial. | KC917239 | KC917239.1 |
| and mixtures of the above. | | |

The gene sequence of the 16S mitochondrial gene of *Homo sapiens* is encoded by the sequence accessible on GenBank under GenBank accession no. NC_012920 AC_00002, version NC_012920.1.

The advantage of using a plurality of gene sequences is to evaluate which regions are the most conserved intra-species and which ones are the most variable among species, so as to direct the design of the primers in those regions.

### Diagnostic kit according to the invention

A further object of the present invention is a diagnostic kit suitable for screening species belonging to the family *Demodex spp.* The kit is usable for carrying out the screening method according to the invention.

The kit comprises

- a sterile medical swab suitable for collecting a biological sample from the skin or the scalp,

- a sterile test tube and, if necessary, a buffer for immersing the swab after sample collection,

- a pair of primers for the amplification of a sequence of *Demodex spp* genetic material, the pair of primers being preferably the one encoded by the sequences SEQ ID No. 1 and 11.

Preferably, the kit of the invention further comprises instructions for performing the method by a user.

Optionally, the kit of the invention further comprises materials or means for genotyping, and/or materials or means for DNA extraction, and/or materials or means for RT-PCR analysis.

These diagnostic kits can be easily used by a patient or by an experienced operator such as a biologist or health technician.

### EXAMPLES

In the following, the Applicant gives examples for illustrative and non-limiting purpose only of the application of the screening method object of the present invention, of the primers and their synthesis process.

### Example 1 - Experimental study

#### 1.1 Experimental method

The samples (18) were collected from the scalp and skin of volunteers. The sample was taken using a swab.

The method involves the use of a collection method that uses a standard laboratory swab. The swab is rubbed vigorously for at least 20 seconds at the collection point (skin or scalp), which is represented by an area of about 1 cm$^2$. After collection, the swab is deposited in a sterile test tube. A buffer can optionally be added to the test tube. At the same time, a traditional sampling was carried out on an area equivalent to that swabbed (1 cm$^2$), using a comedo extractor as described in previous publications [21], in order to carry out a comparative check between the two methods, that of the screening method according to the invention and that of the traditional method. The traditional method of sampling by comedo extractor was chosen as a comparative method to evaluate the efficiency of the method object of the invention as it turned out to be the most efficient method based on the scientific literature [21].

The samples collected using a swab were processed according to the screening method of the invention mentioned above, whereas the samples collected with the comedo extractor were subjected to analysis by conventional microscopy for mite counting.

The DNA genetic material was extracted using the GeneJET Genomic DNA Purification Kit, Thermofisher Scientific, Maryland USA; protocol HS-DNA Quant-it ™ dsDNA HS Assay Kit on the Qubit ™ fluorometer, Invitrogen, California USA).

The amplification conditions for RT-PCR analysis are as follows:

- 12.5 μl of iQ SYBR Green Supermix™;
- 350 nmol forward primer;
- 350 nmol reverse primer;
- 10 ng of DNA;
- $H_2O$ up to 25 μl.

The cycling parameters used for RT-PCR analysis are as follows:

- Denaturation step - temperature of 95°C for 10 seconds;
- Alignment (annealing) and extension step - temperature of 60°C for 30 seconds;
- 40 cycles;
- Melting Curve (or dissociation curve) at temperatures comprised between 60°C and 97°C (reading every 1 second at 1°C temperature range).

For the purposes of the invention, the melting curve tracks the change in fluorescence observed when double-stranded DNA (dsDNA) with embedded dye molecules dissociates, or "melts" into single-stranded DNA (ssDNA) as the temperature of the reaction increases. For example, when double-stranded DNA bound to SYBR® Green dye is heated, a sudden decrease in fluorescence is detected as soon as the melting point (Tm) is reached, due to the dissociation of the DNA strands and subsequent release of the dye. Fluorescence is plotted on a graph against (vs.) temperature; the ratio $-\Delta F/\Delta T$ (fluorescence change/temperature change) is plotted against temperature to obtain a clear view of the melting dynamics.

The pair of primers used is the one encoded by the sequences SEQ ID No. 1 and 11.

Said melting or dissociation curve makes it possible to verify that only one amplified is present in the reaction, which is identifiable because it has a dissociation temperature (Tm or temperature at which the two DNA strands are completely separated due to the breaking of the hydrogen points between the nitrogenous bases), which is recognisable because it depends on the length and composition of the amplicon.

The collection method used collects biological material deriving from the flaking of the stratum corneum of the skin; therefore, the collection could also contain biological material deriving from the presence of *Demodex spp* if this were present. The DNA extracted from the sample will then be a mixture of the two and the relative proportion of mtDNA (mitochondrial DNA) of *Demodex spp* will be in relation to the signal detected (Ct) by the analysis. Since the DNA input used for the analysis is constant (10 ng) and since, as mentioned, the Ct signal is inversely proportional to the amount of the sequence under investigation, lower Ct values will correspond to a higher amount of *Demodex spp* DNA in the sample.

Table 1 shows the Ct values relating to the presence of mtDNA of *Demodex spp* in the samples analysed according to the method of the invention.

Table 1

| Sample | Ct |
|---|---|
| 1 | NEG |
| 2 | NEG |
| 3 | 37.64 |
| 4 | 37.4 |
| 5 | NEG |
| 6 | 29.44 |
| 7 | NEG |
| 8 | 29.95 |
| 9 | 23.54 |
| 10 | 19.37 |
| 11 | 30.6 |
| 12 | NEG |

(continued)

| Sample | Ct |
|---|---|
| 13 | NEG |
| 14 | 23.5 |
| 15 | 31.11 |
| 16 | NEG |
| 17 | NEG |
| 18 | NEG |

*1.2 Comparison of the methods for the quantification of Demodex spp*

A comparison between the screening method object of this invention and the traditional method of counting the mites *Demodex spp* by microscopy was considered in order to verify the sensitivity of the two different methods.

The results obtained on the samples referring to the same subject, relating to the samples analysed with the two methods, the one according to the molecular screening method of the invention and the traditional one, were then compared in parallel (Table 2).

Table 2

| Sample | Ct - Method of Invention | Number of *Demodex spp.* - Traditional method |
|---|---|---|
| 1 | NEG | NEG |
| 2 | NEG | NEG |
| 3 | 37.64 | NEG |
| 4 | 37.40 | NEG |
| 5 | NEG | NEG |
| 6 | 29.44 | 5 |
| 7 | NEG | NEG |
| 8 | 29.95 | 2 |
| 9 | 23.54 | 6 |
| 10 | 19.37 | 14 |
| 11 | 30.60 | NEG |
| 12 | NEG | NEG |
| 13 | NEG | NEG |
| 14 | 23.50 | 2 |
| 15 | 31.11 | 1 |
| 16 | NEG | NEG |
| 17 | NEG | NEG |
| 18 | NEG | NEG |

The results show that the molecular screening method object of the invention has a higher sensitivity than the traditional method; for some samples (for example samples no. 3, 4, 11), where the microscopic analysis did not detect the presence of *Demodex spp,* they were nevertheless found to be positive by molecular analysis using the method object of the invention.

This result is in line with what can be assumed by knowing the biology of *Demodex spp. Demodex folliculorum* localises mainly in the hair follicle, whereas *Demodex brevis* has a more internal localisation, particularly in the lumen of the sebaceous glands; in this respect, mites of the *Demodex brevis* species may not be efficiently collected using the comedo extractor. On the contrary, the method used in the described invention collects the material present on the surface of the skin and of the scalp; moreover, the RT-PCR analysis method has a very high sensitivity in detecting the DNA that could originate both from any mites collected, and from the residue of the tissues deriving from the life cycle of *Demodex* (tissue fragments from dead individuals, eggs, etc.) that are brought to the surface with the sebaceous secretions. The quantity of this biological material, which cannot be detected by microscopy, is however present in relative proportion to the size of the infestation.

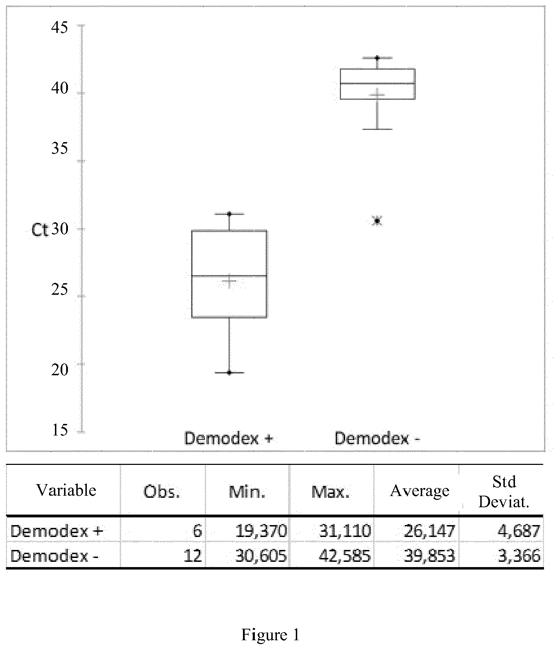

By analysing the Ct values relating to the group of samples that tested positive for the presence of *Demodex spp* by the microscopic method, compared to the group of samples that tested negative (see Figure 1), it can be observed that the average value is significantly lower, thus relative to a higher amount of *Demodex spp* mtDNA detected (Mann-Whitney test / Two-tailed test P<0.0001).

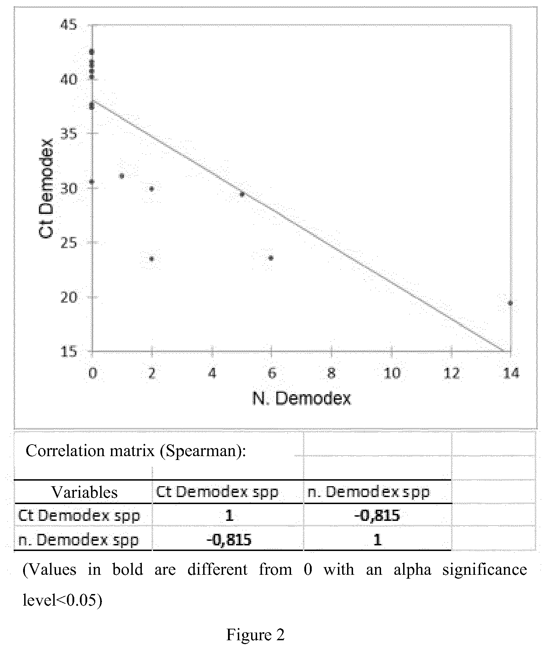

Figure 2 also shows that, despite the different sensitivity of the compared methods, there is a significant correlation between the measurements made with the two methods.

The comparison between the two methods, the one according to the invention and the traditional one, demonstrates that the detection of DNA is correlated with the traditional counting methods. In detail, it was demonstrated that the value of Ct, i.e. of DNA detected, in the samples that tested positive in the microscopic analysis, where the presence of *Demodex spp* was thus detected, is significantly higher than in the samples that tested negative (and vice versa). Therefore, where *Demodex spp* was not detected in the microscopic analysis according to the traditional method, the DNA value is also significantly lower, and vice versa.

*1.3 Conclusions*

The screening method or molecular method object of this invention shows a higher sensitivity than traditional methods and turns out to be a fast, painless and versatile method for the detection of *Demodex spp* in the skin and scalp.

***Example 2 - Specific parameters of direct (forward) and reverse (reverse) primers***

Below Tables 3 and 4 show the output parameters of the Primer-Blast software used to design the pairs of direct and reverse primer.

Table 3 (Length of the amplicon equal to 159)

|  | Direct primer (forward) | Reverse primer |
|---|---|---|
| Sequence (5'-3') | TGCTGCGGTATTTTGACTGTG | TGGGGTCTTCTCGTCTTTAGG |
| Filament Template | Plus | Minus |
| Length | 21 | 21 |
| Start | 20 | 178 |
| Stop | 40 | 158 |
| Tm | 59.46 | 58.82 |
| GC% | 47.62 | 52.38 |
| SC | 3.00 | 2.00 |
| S3C | 1.00 | 1.00 |

Table 4

| Couple | Sequence (5'-3') | Start | Stop | Tm | GC% | SC | S3C |
|---|---|---|---|---|---|---|---|
| I | Direct primer:<br><br>TTGCTGCGGTATTTTGACTG | 19 | 20 | 59.87 | 45.00 | 3 | 1 |
| | Reverse primer:<br><br>TTTGGGGTCTTCTCGTCTTT<br><br>Amplicon length: 162 | 180 | 20 | 58.76 | 45.00 | 2 | 0 |
| II | Direct primer:<br><br>TTGCTGCGGTATTTTGACTG | 19 | 20 | 59.87 | 45.00 | 3 | 1 |
| | Reverse primer:<br><br>TTTTGGGGTCTTCTCGTCTT<br><br>Amplicon length: 163 | 181 | 20 | 58.76 | 45.00 | 2 | 0 |
| III | Direct primer:<br>TTGCTGCGGTATTTTG ACTG | 19 | 20 | 59.87 | 45.00 | 3 | 1 |
| | Reverse primer:<br><br>GGGGTCTTCTCGTCTTTAGGA<br><br>Amplicon length: 159 | 177 | 21 | 59.70 | 52.38 | 3 | 3 |
| IV | Direct primer:<br><br>TTGCTGCGGTATTTTGACTG | 19 | 20 | 59.87 | 45.00 | 3 | 1 |
| | Reverse primer:<br><br>AAAATCAATCTTTCCCCCAAA<br><br>Amplicon length: 216 | 234 | 21 | 59.65 | 33.33 | 4 | 1 |
| V | Direct primer:<br><br>TTGCTGCGGTATTTTGACTG | 19 | 20 | 59.87 | 45.00 | 3 | 1 |
| | Reverse primer:<br><br>AAAGATTTTGGGGTCTTCTCG<br><br>Amplicon length: 168 | 186 | 21 | 59.58 | 42.86 | 4 | 2 |

(continued)

| Couple | Sequence (5'-3') | Start | Stop | Tm | GC% | SC | S3C |
|---|---|---|---|---|---|---|---|
| VI | Direct primer: TTGCTGCGGTATTTTGACTG | 19 | 20 | 59.87 | 45.00 | 3 | 1 |
| | Reverse Primer: GATTTTGGGGTCTTCTCGTC Amplicon length: 165 | 183 | 20 | 58.57 | 50.00 | 2 | 1 |
| VII | Direct primer: TTGCTGCGGTATTTTGACTG | 19 | 20 | 59.87 | 45.00 | 3 | 1 |
| | Reverse primer: GATTTTGGGGTCTTCTCGTCT Amplicon length: 165 | 183 | 21 | 59.57 | 47.62 | 2 | 0 |
| VIII | Direct primer: TTGCTGCGGTATTTTGACTG | 19 | 20 | 59.87 | 45.00 | 3 | 1 |
| | Reverse primer: AGATTTTGGGGTCTTCTCGTC Amplicon length: 166 | 184 | 21 | 59.57 | 47.62 | 3 | 1 |

For the purposes of the invention, self-complementarity (or "SC") means the probability that the primer binds to itself and to the other primer of the pair; whereas, self-3'-complementarity (or "S3C") means the probability that the primer binds to itself and to the other primer of the pair at the end 3'. Ideally, one should choose the primers that have a low self-complementarity and self-3'-complementarity score.

**REFERENCES**

[1] Desch C., Nutting W.B.: "Demodex folliculorum (Simon) and D. brevis Akbulatova of man: rede-scription and reevaluation" The J. of Parasitology, 58 (1): 169-177; 1972;

[2] Nutting B.W., 1981. Demodicosis of ophthalmic concern. American J. of Ophthalmology, 91: 362-372;

[3] Desch C.E., 1989. The digestive system of Demodex folliculorum (Acari:Demodicidae) of man: a light and electron microscope study. Progress in acarology, 1:187-195;

[4] Spickett S.G., 1961. Studies on Demodex folliculorum Simon (1842). Parasitology, 51:181-192;

[5] Principato M., 1994. Demodecose humaine chez des sujets atteints d'alopécie séborrhéique: modifications périodiques dans le cycle biologique des parasites et considérations cliniques sur les causes de la calvitie. Bull. de la Soc.Fran9. de Parasitol., 12(1): 81-91;

[6] Norn MS. Demodex folliculorum. Incidence, regional distribution, pathogenicity. Dan Med Bull. 1971; 18:14-7;

[7] Rufli T, Mumcuoglu Y. The hair follicle mites Demodex folliculorum and Demodex brevis: Biology and medical importance. A review. Dermatologica. 1981; 162:1-11;

[8] El Shazly AM, Ghaneum BM, Morsy TA, Aaty HE. The pathogenesis of Demodex follicularum (hair follicular mites) in females with and without rosacea. J Egypt SOC Parasitol. 2001; 31:867-75;

[9] Georgala S, Katoulis AC, Kylafis GD, Koumantaki-Mathioudaki E, Georgala C, Aroni K. Increased density of Demodex folliculorum and evidence of delayed hypersensitivity reaction in subjects with papulo pustulor Rosacea. J Eur Acad Dermatol Venereol. 2001; 15:441-4;

[10] Vollmer RT. Demodex-associated folliculitis. Am J Dermatopathol. 1996; 18:589-91;

[11] Karincaoglu Y, Bayram N, Aycan O, Esrefoglu M. The clinical importance of Demodex folliculorum presenting with nonspecific facial signs and symptoms. J Dermatol. 2004; 31: 618-26;

[12] Pallotta S, Cianchini G, Martelloni E, Ferranti G, Girardelli CR, Di Lella G, et al. Unilateral demodicidosis. Eur J Dermatol. 1998;8: 191-2;

[13] Bikowski JB, Del Rosso JQ. Demodex Dermatitis: A retrospective analysis of clinical diagnosis and successful treatment with topical crotamiton. Clin Aesthet Dermatol. 2009; 2:20-5;

[14] Whiting DA. Diagnostic and predictive value of horizontal sections of scalp biopsy specimen in male pattern androgenetic alopecia. J Am Acad Dermatol. 1993; 28:755-63;

[15] Millikan LE. Androgenetic alopecia: The role of inflammation and Demodex. Int J Dermatol. 2001; 40:475-6;

[16] Jaworsky C, Kligman AM, Murphy GF. Characterization of inflammatory infiltrates in male pattern androgenetic alopecia: Implication for pathogenesis. Br J Dermatol. 1992; 127:239-46;

[17] Forton F, Seys B. Density of Demodex folliculorum in rosacea: A case-control study using standardized skin-surface biopsy. Br J Dermatol. 1993; 128:650-9;

[18] Marks R, Dawber RP. Skin surface biopsy: An improved technique for the examination of the horny layer. Br J Dermatol. 1971; 84:117-23;

[19] Forton F, Song M. Limitations of standardized skin surface biopsy in measurement of the density of Demodex folliculorum: A case report. Br J Dermatol. 1998; 139:697-700;

[20] Rather PA, Hassan I. Human demodex mite: the versatile mite of dermatological importance. Indian J Dermatol. 2014 Jan; 59(1):60-6;

[21] Yun CH, Yun JH, Baek JO, Roh JY, Lee JR. Demodex Mite Density Determinations by Standardized Skin Surface Biopsy and Direct Microscopic Examination and Their Relations with Clinical Types and Distribution Patterns. Ann Dermatol. 2017 Apr;29(2):137-142.

**Claims**

1. Screening method of mite species of *Demodex spp* comprising the steps of

a) preparing a sample of biological material for which it is intended to verify the presence of *Demodex spp* genetic material;

b) extracting the genetic material from the sample of biological material prepared;

c) conducting a quantitative PCR analysis of the extracted genetic material, using a pair of primers synthesised for the amplification of a pre-determined sequence of *Demodex spp* genetic material

wherein the pre-determined sequence of *Demodex spp* genetic material is a nuclear DNA or mitochondrial DNA sequence of *Demodex spp.*

2. Screening method according to claim 1, wherein the pre-determined sequence of *Demodex spp* genetic material is a homologous sequence of the 16S mitochondrial gene of *Demodex spp.*

3. Screening method according to claim 1 or 2, wherein the sample of biological material is a skin and/or scalp sample.

4. Screening method according to any one of claims from 1 to 3, wherein the mite species of *Demodex spp* comprise or consist of *Demodex folliculorum* and/or *Demodex brevis.*

5. Screening method according to any one of claims from 1 to 4, wherein the PCR analysis is **characterised by** the generation of amplicons having a length comprised between 100 and 220 bp.

6. Screening method according to any one of claims from 1 to 5, wherein a threshold cycle (Ct) of the PCR analysis < about 37.65 is indicative of positivity to *Demodex spp.*

7. Screening method according to any one of claims from 1 to 6, wherein the pair of primers comprises a direct primer encoded by the sequence *N*TGCTGCGGTATTTTGACTG*NN* (SEQ ID No. 12), wherein *N* in position 1 and *N* in position 21 are independently chosen from: abasic site, or Thymine (T); and *N* in position 22 is an abasic site, or Guanine (G).

8. Screening method according to any one of claims from 1 to 7, wherein the pair of primers comprises a reverse primer encoded by the sequence chosen from the group consisting of:

- *NN*TTTGGGGTCTTCTCGTCTT*N* (SEQ ID No. 13), wherein *N* in position 1, *N* in position 2 and *N* in position 22 are independently chosen from: abasic site, or Thymine (T);
- *NNN*GATTTTGGGGTCTTCTCG*NNN* (SEQ ID No. 14), wherein *N* in position 1, *N* in position 2, *N* in position 3 are independently chosen from: abasic site, or Adenosine (A); *N* in position 22 and *N* in position 24 are independently chosen from: abasic site, or Thymine (T); *N* in position 23 is an abasic site, or Cytosine (C);
- *N*GGGGTCTTCTCGTCTTTAGG*N* (SEQ ID No. 15), wherein *N* in position 1 is an abasic site, or Thymine (T), and *N* in position 22 is an abasic site, or Adenosine (A); and
- AAAATCAATCTTTCCCCCAAA (SEQ ID No. 6).

9. Pair of primers for the amplification of a sequence of *Demodex spp* genetic material, comprising a direct primer encoded by the sequence NTGCTGCGGTATTTTGACTGNN (SEQ ID No. 12), wherein *N* in position 1 and *N* in position 21 are independently chosen from: abasic site, or Thymine (T); and *N* in position 22 is an abasic site, or Guanine (G).

10. Pair of primers according to claim 9, comprising a reverse primer encoded by the sequence chosen from the group consisting of:

- *NN*TTTGGGGTCTTCTCGTCTT*N* (SEQ ID No. 13), wherein *N* in position 1, *N* in position 2 and *N* in position 22 are independently chosen from: abasic site, or Thymine (T);
- *NNN*GATTTTGGGGTCTTCTCG*NNN* (SEQ ID No. 14), wherein *N* in position 1, *N* in position 2, *N* in position 3 are independently chosen from: abasic site, or Adenosine (A); *N* in position 22 and *N* in position 24 are independently chosen from: abasic site, or Thymine (T); *N* in position 23 is an abasic site, or Cytosine (C);
- *N*GGGGTCTTCTCGTCTTTAGG*N* (SEQ ID No. 15), wherein *N* in position 1 is an abasic site, or Thymine (T), and *N* in position 22 is an abasic site, or Adenosine (A); and
- AAAATCAATCTTTCCCCCAAA (SEQ ID No. 6).

11. Kit for screening mite species of *Demodex spp* comprising:

- a sterile medical swab suitable for collecting a biological sample from the skin or the scalp,
- a sterile test tube and, if necessary, a buffer for immersing the swab after sample collection,
- a pair of primers for the amplification of a sequence of *Demodex spp* genetic material, comprising a primer according to claim 9 or 10.

| Variable | Obs. | Min. | Max. | Average | Std Deviat. |
|---|---|---|---|---|---|
| Demodex + | 6 | 19,370 | 31,110 | 26,147 | 4,687 |
| Demodex - | 12 | 30,605 | 42,585 | 39,853 | 3,366 |

Figure 1

Correlation matrix (Spearman):

| Variables | Ct Demodex spp | n. Demodex spp |
|---|---|---|
| Ct Demodex spp | 1 | -0,815 |
| n. Demodex spp | -0,815 | 1 |

(Values in bold are different from 0 with an alpha significance level<0.05)

Figure 2

Europäisches
Patentamt

European
Patent Office

Office européen
des brevets

# EUROPEAN SEARCH REPORT

Application Number

EP 22 18 3165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KASETSUWAN NGAMJIT ET AL: "Prevalence of ocular demodicosis among patients at Tertiary Care Center, Bangkok, Thailand", INTERNATIONAL JOURNAL OF OPHTHALMOLOGY , 18 January 2017 (2017-01-18), XP055902642, CN ISSN: 2222-3959, DOI: 10.18240/ijo.2017.01.20 Retrieved from the Internet: URL:https://www.ncbi.nlm.nih.gov/pmc/articles/PMC5225360/pdf/ijo-10-01-122.pdf | 1,4-6 | INV. C12Q1/6888 |
| Y | * page 123, right-hand column * | 2,3 | |
| X | YA-E ZHAO ET AL: "Phylogenetic relationships inmites (Acari: Demodicidae) based on mitochondrial 16S rDNA partial sequences", PARASITOLOGY RESEARCH ; FOUNDED AS ZEITSCHRIFT FÜR PARASITENKUNDE, SPRINGER, BERLIN, DE, vol. 111, no. 3, 13 May 2012 (2012-05-13), pages 1113-1121, XP035104557, ISSN: 1432-1955, DOI: 10.1007/S00436-012-2941-7 | 9-11 | |
| Y | * page 1114, right-hand column, last paragraph; figure 1 * | 2,7,8 | |
| X | PRASHER PAWAN ET AL: "Molecular identification and phylogenetic relationship of Demodex mites based on mitochondrial 16S rDNA", TROPICAL PARASITOLOGY, vol. 10, no. 2, 1 July 2020 (2020-07-01), pages 136-141, XP055902839, ISSN: 2229-5070, DOI: 10.4103/tp.TP_76_19 | 9-11 | |
| Y | * page 2, last paragraph – page 3, paragraph 2 * | 2,7,8 | |

TECHNICAL FIELDS
SEARCHED (IPC)

C12Q

-/--

The present search report has been drawn up for all claims

| Place of search | Date of completion of the search | Examiner |
|---|---|---|
| The Hague | 16 January 2023 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 18 3165

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CASAS CHRISTIANE ET AL: "Quantification of Demodex folliculorum by PCR in rosacea and its relationship to skin innate immune activation", EXPERIMENTAL DERMATOLOGY, vol. 21, no. 12, 22 November 2012 (2012-11-22), pages 906-910, XP055902906, COPENHAGEN; DK ISSN: 0906-6705, DOI: 10.1111/exd.12030 * page 907, right-hand column * ----- | 3 | |

TECHNICAL FIELDS
SEARCHED (IPC)

The present search report has been drawn up for all claims

| Place of search | Date of completion of the search | Examiner |
|---|---|---|
| The Hague | 16 January 2023 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- DNA Amplification by the Polymerase Chain Reaction: Providing a Revolutionary Method for All Biologists. **MARJORIE A.HOY.** Insect Molecular Genetics (Fourth Edition), An Introduction to Principles and Applications. 2019, 263-314
- **DESCH C. ; NUTTING W.B.** Demodex folliculorum (Simon) and D. brevis Akbulatova of man: rede-scription and reevaluation. *The J. of Parasitology,* 1972, vol. 58 (1), 169-177
- **NUTTING B.W.** Demodicosis of ophthalmic concern. *American J. of Ophthalmology,* 1981, vol. 91, 362-372
- **DESCH C.E.** The digestive system of Demodex folliculorum (Acari:Demodicidae) of man: a light and electron microscope study. *Progress in acarology,* 1989, vol. 1, 187-195
- **SPICKETT S.G.** Studies on Demodex folliculorum Simon (1842). *Parasitology,* 1961, vol. 51, 181-192
- **PRINCIPATO M.** Demodecose humaine chez des sujets atteints d'alopécie séborrhéique: modifications périodiques dans le cycle biologique des parasites et considérations cliniques sur les causes de la calvitie. *Bull. de la Soc.Fran9. de Parasitol.,* 1994, vol. 12 (1), 81-91
- **NORN MS.** Demodex folliculorum. Incidence, regional distribution, pathogenicity. *Dan Med Bull.,* 1971, vol. 18, 14-7
- **RUFLI T ; MUMCUOGLU Y.** The hair follicle mites Demodex folliculorum and Demodex brevis: Biology and medical importance. A review. *Dermatologica.,* 1981, vol. 162, 1-11
- **EL SHAZLY AM ; GHANEUM BM ; MORSY TA ; AATY HE.** The pathogenesis of Demodex follicularum (hair follicular mites) in females with and without rosacea. *J Egypt SOC Parasitol.,* 2001, vol. 31, 867-75
- **GEORGALA S ; KATOULIS AC ; KYLAFIS GD ; KOUMANTAKI-MATHIOUDAKI E ; GEORGALA C ; ARONI K.** Increased density of Demodex folliculorum and evidence of delayed hypersensitivity reaction in subjects with papulo pustulor Rosacea. *J Eur Acad Dermatol Venereol.,* 2001, vol. 15, 441-4
- **VOLLMER RT.** Demodex-associated folliculitis. *Am J Dermatopathol.,* 1996, vol. 18, 589-91

- **KARINCAOGLU Y ; BAYRAM N ; AYCAN O ; ESREFOGLU M.** The clinical importance of Demodex folliculorum presenting with nonspecific facial signs and symptoms. *J Dermatol.,* 2004, vol. 31, 618-26
- **PALLOTTA S ; CIANCHINI G ; MARTELLONI E ; FERRANTI G ; GIRARDELLI CR ; DI LELLA G et al.** Unilateral demodicidosis. *Eur J Dermatol.,* 1998, vol. 8, 191-2
- **BIKOWSKI JB ; DEL ROSSO JQ.** Demodex Dermatitis: A retrospective analysis of clinical diagnosis and successful treatment with topical crotamiton. *Clin Aesthet Dermatol.,* 2009, vol. 2, 20-5
- **WHITING DA.** Diagnostic and predictive value of horizontal sections of scalp biopsy specimen in male pattern androgenetic alopecia. *J Am Acad Dermatol.,* 1993, vol. 28, 755-63
- **MILLIKAN LE.** Androgenetic alopecia: The role of inflammation and Demodex. *Int J Dermatol.,* 2001, vol. 40, 475-6
- **JAWORSKY C ; KLIGMAN AM ; MURPHY GF.** Characterization of inflammatory infiltrates in male pattern androgenetic alopecia: Implication for pathogenesis. *Br J Dermatol.,* 1992, vol. 127, 239-46
- **FORTON F ; SEYS B.** Density of Demodex folliculorum in rosacea: A case-control study using standardized skin-surface biopsy. *Br J Dermatol.,* 1993, vol. 128, 650-9
- **MARKS R ; DAWBER RP.** Skin surface biopsy: An improved technique for the examination of the horny layer. *Br J Dermatol.,* 1971, vol. 84, 117-23
- **FORTON F ; SONG M.** Limitations of standardized skin surface biopsy in measurement of the density of Demodex folliculorum: A case report. *Br J Dermatol.,* 1998, vol. 139, 697-700
- **RATHER PA ; HASSAN I.** Human demodex mite: the versatile mite of dermatological importance. *Indian J Dermatol.,* January 2014, vol. 59 (1), 60-6
- **YUN CH ; YUN JH ; BAEK JO ; ROH JY ; LEE JR.** Demodex Mite Density Determinations by Standardized Skin Surface Biopsy and Direct Microscopic Examination and Their Relations with Clinical Types and Distribution Patterns. *Ann Dermatol,* April 2017, vol. 29 (2), 137-142